# EUROPÄISCHE PATENTSCHRIFT

(11) **EP 2 984 067 B1**
(45) Veröffentlichungstag und Bekanntmachung des Hinweises auf die Patenterteilung: **25.10.2017**
(21) Anmeldenummer: 14715615.2
(22) Anmeldetag: 08.04.2014
(51) Int. Cl.: C07C 209/78, B01D 17/02, B01D 17/04, C07C 263/10

(54) **VERFAHREN ZUR HERSTELLUNG VON DI- UND POLYAMINEN DER DIPHENYLMETHANREIHE**
METHOD FOR THE PREPARATION OF DI- AND POLYAMINES OF THE DIPHENYL METHANE SERIES
PROCÉDÉ DESTINÉ À LA FABRICATION DE DI- ET POLYAMINES DE LA SÉRIE DIPHÉNYLMÉTHANE

(30) Priorität: 11.04.2013 EP 13163357
(43) Veröffentlichungstag der Anmeldung: 17.02.2016
(73) Patentinhaber: Covestro Deutschland AG, 51373 Leverkusen (DE)
(72) Erfinder: KNAUF, Thomas, 41542 Dormagen (DE); LORENZ, Wolfgang, 41540 Dormagen (DE); WERSHOFEN, Stefan, 41065 Mönchengladbach (DE); ADAMSON, Richard, 42799 Leichlingen (DE); BECKER, Karsten, 51371 Leverkusen (DE)
(74) Vertreter: Levpat
(86) Internationale Anmeldenummer: PCT/EP2014/056979
(87) Internationale Veröffentlichungsnummer: WO 2014/166901

(56) Entgegenhaltungen:
- EP-A1- 2 103 595
- WO-A1-02/053601
- WO-A1-2012/104769
- SULZER: 'Liquid-Liquid Separation Technology', [Online] Seiten 1 - 16 Sulzer Chemtech Gefunden im Internet: <URL:http://www.sulzer.com/as/-/media/Docum ents/ProductsAndServices/Separation_Technol ogy/Coalescers/Brochures/Liquid_Liquid_Sepa ration_Technology.pdfwww.scribd.com/documen t/138647988/Liquid-Liquid-Separation-Techno logywww.sulzer.com> [gefunden am 2016-11-14]

## Beschreibung

Gegenstand der vorliegenden Erfindung ist ein Verfahren zur Herstellung von Di- und Polyaminen der Diphenylmethanreihe, bei dem durch Einsatz eines Koaleszenz-Hilfsmittels in der Phasentrennung des in der Wäsche des neutralisierten Rohprodukts erhaltenen Verfahrensprodukts der Anteil an Wasser und damit auch an wasserlöslichen Verunreinigungen in der organischen, MDA enthaltenden Phase verringert wird. Als Koaleszenz-Hilfsmittel wird erfindungsgemäß ein Filterbett aus Koaleszenz-Faser-Material eingesetzt. Die nach destillativer Reinigung dieser MDA enthaltenden organischen Phase gewonnenen Di- und Polyamine der Diphenylmethanreihe eignen sich hervorragend zur Herstellung der entsprechenden Isocyanate.

Die Herstellung von Di- und Polyaminen der Diphenylmethanreihe (MDA) durch Umsetzung von Anilin mit Formaldehyd in Gegenwart saurer Katalysatoren ist allgemein bekannt. Im Sinne der vorliegenden Erfindung werden unter Di- und Polyaminen der Diphenylmethanreihe Amine und Gemische von Aminen folgenden Typs verstanden:

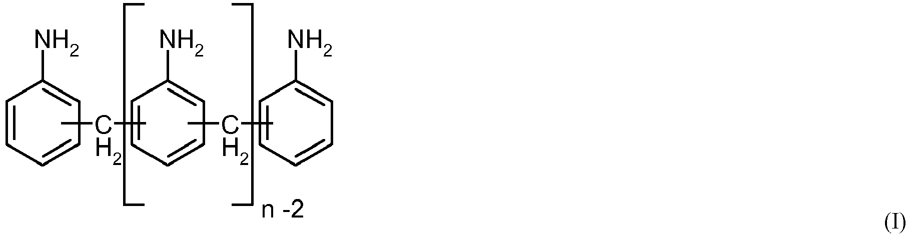

Dabei steht n für eine natürliche Zahl ≥ 2. Im Folgenden werden die Verbindungen dieses Typs, bei denen n = 2 ist, als *Diamine der Diphenylmethanreihe* oder *Diaminodiphenylmethane* (nachfolgend MMDA) bezeichnet. Verbindungen dieses Typs, bei denen n > 2 ist, werden im Rahmen dieser Erfindung als *Polyamine der Diphenylmethanreihe* oder *Polyphenylenpolymethylenpolyamine* (nachfolgend PMDA) bezeichnet. Gemische aus beiden Typen werden als *Di- und Polyamine der Diphenylmethanreihe* bezeichnet (nachfolgend MDA). Die korrespondierenden Isocyanate, die sich formal durch Ersatz aller NH₂-Gruppen durch NCO-Gruppen aus den Verbindungen der Formel (I) ableiten lassen, werden dementsprechend als *Diisocyanate der Diphenylmethanreihe* (nachfolgend MMDI), *Polyisocyanate der Diphenylmethanreihe* oder *Polyphenylenpolymethylenpolyisocyanate* (nachfolgend PMDI) bzw. *Di- und Polyisocyanate der Diphenylmethanreihe* (nachfolgend MDI) bezeichnet. Das Polymere (n > 2) liegt dabei sowohl beim Amin als auch beim Isocyanat in der Regel immer im Gemisch mit dem Dimeren (n = 2) vor, so dass in der Praxis nur zwei Verbindungstypen relevant sind, die reinen Dimeren (MMDA bzw. MMDI) und das Gemisch aus Dimeren und Polymeren (MDA bzw. MDI).

Industriell werden die Di- und Polyamingemische überwiegend durch Phosgenierung zu den entsprechenden Di- und Polyisocyanaten der Diphenylmethanreihe umgesetzt. Die kontinuierliche oder teilweise diskontinuierliche Herstellung von MDA ist z. B. in US-A-5286760, EP-A-451442 und WO-A-99/40059 offenbart.

Die Aufarbeitung des in der Herstellung erhaltenen sauren Reaktionsgemisches wird gemäß dem Stand der Technik durch Neutralisation mit einer Base eingeleitet. Nach dem Stand der Technik erfolgt die Neutralisation üblicherweise bei Temperaturen von beispielsweise 90 °C bis 100 °C ohne Zusatz weiterer Substanzen. (H.J. Twitchett, Chem. Soc. Rev. 3(2), S. 223 (1974)). Sie kann aber auch auf einem anderen Temperaturniveau erfolgen, um z. B. den Abbau von störenden Nebenprodukten zu beschleunigen. Hydroxide der Alkali- und Erdalkalielemente sind als Basen geeignet. Vorzugsweise kommt wässrige NaOH zum Einsatz.

Im Anschluss an die Neutralisation wird in einem Trennbehälter die organische von der wässrigen Phase getrennt. Die nach Abtrennung der wässrigen Phase verbleibende Roh-MDA enthaltende organische Phase wird weiteren Aufarbeitungsschritten unterzogen, wie z. B. einer Wäsche mit Wasser (Basenwäsche), um Restsalze aus dem Roh-MDA zu waschen. Abschließend wird das so gereinigte Roh-MDA von überschüssigem Anilin, Wasser und anderen im Gemisch vorhandenen Stoffen (z. B. weiteren Lösungsmitteln) durch geeignete Verfahren wie z. B. Destillation, Extraktion oder Kristallisation befreit. Insbesondere können zwei- oder mehrstufige Destillationen eingesetzt werden. Bei der Dampf/Flüssigkeitstrennung in der Voreindampfung im Falle einer solchen zwei- oder mehrstufigen Destillation kann der Mitriss von MDA-haltigen Tröpfchen in die Dampfphase durch einen dem Fachmann bekannten Verfahrensschritt zur Tropfenabscheidung reduziert werden, z. B. durch den Einbau von Schwerkraftabscheidern, Zentrifugalabscheidern wie z. B. Zyklonen, Prallabscheidern bzw. Umlenkabscheidern, wie z. B. Lamellenabscheidern, Festbetten oder Packungen zur Tropfenabscheidung, Gestrickabscheidern, Venturiabscheidern oder eine Kombination der vorgenannten Mechanismen. Wie aus EP 1 813 598 B1 bekannt, führt ein erhöhter MDA-Anteil im Einsatzanilin zu Qualitätseinbußen im MDA und daraus hergestelltem MDI.

Im gleichen Verfahrensschritt können auch aminische Nebenkomponenten im Anilin, die auch aus dem Anilinverfahren selbst stammen können und die in EP 2 103 595 A1 vorbeschrieben sind, insbesondere die leichtsiedenden ungesättigten und/oder substituierten (cyclo)aliphatischen primären, sekundären oder tertiären Amine wie Cyclohexylamin, N,N-Dicyclohexylamin und N-Methylcyclohexylamin, entfernt werden. Dadurch reduziert sich die erforderliche Menge an HCl in der Protonierung des Aminals, weil diese Nebenkomponenten ansonsten bei der Protonierung mit Anilin konkurrieren können und somit die effektive Protonierung senken.

Die nach dem Stand der Technik übliche Aufarbeitung ist beispielsweise offenbart in EP 1652 835 A1, Seite 3, Zeile 58 bis Seite 4, Zeile 13 und EP 2 103 595 A1, Seite 7, Zeile 21 bis 37. EP 1 652 835 A1 lehrt, dass die Abtrennung der wässrigen Phase von der Produkt enthaltenden organischen Phase nach der Neutralisation und/oder der nachfolgenden Wäsche durch die Ausbildung einer dritten Phase (Mulm bzw. Mulmschicht) sehr stark beeinträchtigt werden kann. Diese dritte Phase ist eine stabile, ggf. voluminöse Zwischenphase, die zwischen der wässrigen und der organischen Phase auftritt und die Phasentrennung erschwert und im Extremfall sogar vollständig verhindert. Im für den betrieblichen Ablauf ungünstigsten Fall müssen der oder die betroffenen Phasentrennbehälter vollständig entleert und gereinigt werden. Der Inhalt des bzw. der Phasentrennbehälter ist dann aufwändig aufzuarbeiten oder zu entsorgen, was mit erheblichen Kosten verbunden ist. Dies kann unter Umständen auch dazu führen, dass die kontinuierliche Produktion unterbrochen werden muss. Zur Lösung dieses Problems schlägt die zitierte Schrift ein Verfahren vor, bei dem die als Katalysator eingesetzte Salzsäure weniger als 0,001 Gewichtsprozent an zwei- und /oder höherwertigen Metallionen enthält.

In WO 2008/148631 A1 wird die gleiche Problematik beobachtet, wobei in diesem Fall vorgeschlagen wird, Formalin, das weniger als 0,001 Gewichtsprozent an zwei- und /oder höherwertigen Metallionen enthält, einzusetzen.

In beiden Patentanmeldungen wird nicht beschrieben, dass neben HCl und Formalin auch die weiteren Edukte der MDA-Herstellung, nämlich Natronlauge und Anilin, ebenfalls zur Phasentrennproblematik und / oder Mulmbildung beitragen können. Des Weiteren wird in den beiden Patentanmeldungen nicht beschrieben, ob die Probleme der Phasentrennung durch Maßnahmen in der Aufarbeitung gelöst werden können.

Wenn sich die Bildung einer Mulmschicht nicht völlig vermeiden lässt, so wird diese schließlich in eine der beiden Phasen gelangen. Gelangt die Mulmschicht in die organische Phase, so ist dies im Fall der Phasentrennung nach der Neutralisation des Rohprodukts weniger bedenklich als im Fall der Phasentrennung nach der Wäsche des neutralisierten Produktes. Denn im Fall der letztgenannten Phasentrennung gelangen dann mit der Mulmschicht nicht nur größere Mengen Wassers, sondern natürlich auch die darin gelösten Stoffe wie z. B. NaOH und NaCl, aus der Neutralisation mit in die weiteren Verarbeitungsschritte, wo sie sich störend auswirken können, z. B. durch Salzablagerungen in Apparaten und Rohrleitungen im Bereich der Destillation. Selbst wenn die Ausbildung einer Mulmschicht (z. B. durch die in EP 1 652 835 A1 und WO 2008/148631 A1 beschriebenen Maßnahmen) verhindert werden kann, kann die nach Phasentrennung erhaltene organische Phase noch beträchtliche Anteile an wässrigen Bestandteilen als disperse Phase enthalten, die in weiteren Verarbeitungsschritten ähnliche Probleme wie eine mitgeschleppte Mulmschicht zur Folge haben können.

EP 2 103 595 A1 offenbart im Zusammenhang mit der Phasentrennung nach der Neutralisation des Rohprodukts, dass diese Phasentrennung durch Zugabe von Wasser und/oder Anilin unterstützt werden kann. Bevorzugt wird das durch Zugabe von Wasser und/oder Anilin verdünnte Reaktionsgemisch in Scheideflaschen mit die Koaleszenz der beiden Phasen unterstützenden Plattenpaketen als Einbauten in eine organische und wässrige Phase getrennt (Absätze [0043] und [0044]). Bei der Phasentrennung nach der Wäsche des neutralisierten Produktes lassen sich mit dieser Vorgehensweise, weil hier, wie zuvor erläutert, die Anforderungen an die Güte der Phasentrennung viel höher sind, keine vollständig zufriedenstellenden Ergebnisse erzielen. Dies wurde in EP 2 103 595 A1 nicht erkannt.

Es wäre daher wünschenswert, verfahrenstechnische Maßnahmen zur Verfügung zu haben, um diese Problematik beherrschen zu können.

WO 02/053601 A1 befasst sich mit einem Verfahren zur Herstellung hochreaktiver Polyisobutene. In diesem Zusammenhang wird der Einsatz von Koaleszens-fördernden Einbauten bei der Trennung von wässrigen und organischen Phasen offenbart.

Der Prospekt *Sulzer Chemtech "Liquid-Liquid Separation Technology"* der Fa. Sulzer stellt verschiedene Trennhilfen vor. Ob und unter welchen Bedingungen solche Trennhilfen in der MDA-Herstellung eingesetzt werden können, ist dem Prospekt nicht zu entnehmen.

Die Güte eines Aufarbeitungsverfahrens zur Herstellung von MDA ist einerseits definiert durch den Gehalt des Produktes an unerwünschten Verunreinigungen, die durch unsachgemäße Reinigungsschritte auftreten. Andererseits ist die Güte eines Aufarbeitungsverfahrens dadurch definiert, dass der gesamte Prozess ohne technischen Produktionsausfall betrieben werden kann.

Den beschriebenen Verfahren des Standes der Technik gelingt es zwar, mit hoher Ausbeute MDA herzustellen, jedoch werden keine technischen Hilfsmittel beschrieben, die die Abtrennung wässriger Bestandteile aus dem gewaschenen neutralisierten Produkt mit der wünschenswerten Wirksamkeit verbessern könnten.

Es bestand also ein Bedarf an einem Verfahren zur Herstellung von Di- und Polyaminen der Diphenylmethanreihe, bei dem es durch einfache Maßnahmen möglich ist, die wässrigen Anteile der nach Phasentrennung des gewaschenen Roh-MDA erhaltenen organischen Phase zu minimieren. Dies würde die Wirtschaftlichkeit bestehender MDA-Prozesse verbessern.

Dem vorstehend Gesagten Rechnung tragend ist ein Gegenstand der vorliegenden Erfindung ein Verfahren zur Herstellung von Di- und Polyaminen der Diphenylmethanreihe, bei dem
a) Anilin und Formaldehyd in Gegenwart eines sauren Katalysators zu einem Reaktionsgemisch enthaltend Di- und Polyamine der Diphenylmethanreihe umgesetzt werden,
b) das Reaktionsgemisch enthaltend Di- und Polyamine der Diphenylmethanreihe neutralisiert wird,
c) das neutralisierte Reaktionsgemisch enthaltend Di- und Polyamine der Diphenylmethanreihe in einem Trennbehälter in eine organische Phase (1) enthaltend Di- und Polyamine der Diphenylmethanreihe und eine wässrige Phase aufgetrennt wird,
d) die organische Phase (**1**) enthaltend Di- und Polyamine der Diphenylmethanreihe in einem Waschbehälter mit Waschflüssigkeit weiter gereinigt wird,
e) das in Schritt d) erhaltene Gemisch von wässrigen Bestandteilen befreit wird, wobei eine organische Phase (**2**) enthaltend Di- und Polyamine der Diphenylmethanreihe erhalten wird,
f) die Di- und Polyamine der Diphenylmethanreihe enthaltende organische Phase (**2**) destillativ von Wasser und Anilin befreit wird,
wobei zur Abtrennung der wässrigen Bestandteile in Schritt e) das in Schritt d) erhaltene Gemisch
e.1) in einem Trennbehälter in eine wässrige und eine organische, noch Restmengen an wässrigen Bestandteilen umfassende, Phase (**2**a) getrennt wird, wobei in der organischen Phase (**2a**) wässrige Bestandteile dispergiert sind, und dann
e.2) die in Schritt e.1) erhaltene organische Phase (**2**a) (i) bei einer Temperatur im Bereich von 50 °C bis 120 °C und bei einem solchen Druck, dass ein Sieden des dispersen Systems unterbleibt, ohne Verwendung von Inertgasen durch ein Filterbett aus Koaleszenz-Faser-Material geführt und (ii) anschließend in eine wässrige Phase und die organische Phase (**2**) getrennt wird.

Die Erfindung betrifft auch ein Verfahren zur Herstellung von Di- und Polyisocyanaten der Diphenylmethanreihe, bei dem Di- und Polyamine der Diphenylmethanreihe nach dem erfindungsgemäßen Verfahren hergestellt werden und anschließend mit Phosgen zu den entsprechenden Di- und Polyisocyanaten umgesetzt werden.

Nachstehend werden Ausführungsformen der Erfindung näher beschrieben. Dabei können verschiedene Ausführungsformen beliebig miteinander kombiniert werden, sofern sich für den Fachmann aus dem Kontext nicht eindeutig das Gegenteil ergibt.

Die säurekatalysierte Kondensation von Anilin und Formaldehyd in **Schritt a)** kann nach einem Verfahren nach dem Stand der Technik durchgeführt werden. Dabei werden bevorzugt Anilin und wässrige Formaldehydlösung bei Molverhältnissen im Bereich von 1,7 : 1 bis 20 : 1, besonders bevorzugt 1,7 : 1 bis 5 : 1 in Gegenwart eines sauren Katalysators, bevorzugt einer starken Mineralsäure wie Salzsäure, bei Einsatz von bevorzugt 0,001 bis 0,9 Mol Mineralsäure pro Mol Anilin, besonders bevorzugt 0,05 bis 0,5 Mol Mineralsäure pro Mol Anilin, kondensiert. Es können auch feste saure Katalysatoren, wie in der Literatur beschrieben, verwendet werden. Dabei kann Formaldehyd in eine Mischung von Anilin und saurem Katalysator gegeben und die Reaktionslösung durch stufenweises Erhitzen ausreagiert werden. Alternativ können auch Anilin und Formaldehyd zunächst vorreagiert werden und anschließend mit oder ohne vorhergehender Wasserabtrennung mit dem sauren Katalysator oder einem Gemisch von weiterem Anilin und saurem Katalysator versetzt werden, wonach die Reaktionslösung durch stufenweises Erhitzen ausreagiert wird. Diese Reaktion kann kontinuierlich oder diskontinuierlich nach einem der zahlreichen in der Literatur beschriebenen Verfahren ausgeführt werden (z. B. in EP 1 616 890 A1 oder EP 127 0544 A1).

In **Schritt b)** wird das Reaktionsgemisch enthaltend die Di- und Polyamine der Diphenylmethanreihe ggf. unter Zusatz von Wasser und / oder Anilin neutralisiert. Nach dem Stand der Technik erfolgt die Neutralisation üblicherweise bei Temperaturen von beispielsweise 90 °C bis 100 °C ohne Zusatz weiterer Substanzen. Sie kann aber auch auf einem anderen Temperaturniveau erfolgen, um z. B. den Abbau von störenden Nebenprodukten zu beschleunigen. Als Basen sind beispielsweise geeignet die Hydroxide der Alkali- und Erdalkalielemente. Vorzugsweise kommt wässrige NaOH zur Anwendung. Die zur Neutralisation eingesetzte Base wird bevorzugt in Mengen von grösser 100 %, besonders bevorzugt 105 % bis 120 % der für die Neutralisation des eingesetzten sauren Katalysators stöchiometrisch benötigten Menge eingesetzt (siehe EP 1 652 835 A1). Die Rohrleitung zwischen NaOH-Zugabe und Neutralisationsreaktor und / oder nachfolgendem Phasentrennapparat ist üblicherweise aus einem gegenüber dem Reaktionsgemisch beständigen Werkstoff gefertigt. Ein zusätzlicher Schutz der Rohrleitung kann durch Einziehen eines Teflon-Inliners erreicht werden.

Anschließend wird in **Schritt c)** das neutralisierte Reaktionsgemisch enthaltend die Di- und Polyamine der Diphenylmethanreihe in eine organische Phase enthaltend Di- und Polyamine der Diphenylmethanreihe und eine wässrige Phase aufgetrennt. Dies kann durch die Zugabe von Anilin und / oder Wasser unterstützt werden. Wird die Phasentrennung durch Zugabe von Anilin und / oder Wasser unterstützt, so erfolgt deren Zugabe bevorzugt bereits unter intensivem Mischen in der Neutralisation. Dabei kann die Vermischung in Mischstrecken mit statischen Mischern, in Rührkesseln oder Rührkesselkaskaden oder aber in einer Kombination von Mischstrecken und Rührkessel erfolgen. Es kann eine Leitfähigkeitsmessung mit Abschaltung in der Neutralisation und Wäsche eingebaut werden, um eine Phasenumkehr zu detektieren und den NaOH-Überschuss sicherzustellen. Der Stand in den Trennbehältern kann durch eine kapazitive Sonde oder durch Schwimmer kontrolliert und geregelt werden. Das neutralisierte und durch Zugabe von Anilin und / oder Wasser verdünnte Reaktionsgemisch wird anschließend bevorzugt einem Apparat zugeführt, der aufgrund seiner Konfiguration und / oder Einbauten besonders zur Auftrennung in eine organische Phase enthaltend MDA und eine wässrige Phase geeignet ist, bevorzugt Phasentrenn- oder Extraktionsvorrichtungen entsprechend dem Stand der Technik, wie sie beispielsweise in Mass-Transfer Operations, 3rd Edition, 1980, McGraw-Hill Book Co, S. 477 bis 541, oder Ullmann's Encyclopedia of Industrial Chemistry (Vol. 21, Liquid-Liquid Extraction, E. Müller et al., Seite 272-274, 2012 Wiley-VCH Verlag GmbH & Co. KGaA, Weinheim, DOI: 10.1002/14356007.b03_06.pub2) oder in Kirk-Othmer Encyclopedia of Chemical Technology (siehe "http://onlinelibrary.wiley.com/book/10.1002/0471238961", Published Online: 15 Juni 2007, Seite 22-23) beschrieben sind (Mixer-Settler-Kaskade oder Absetzbehälter). Eine Optimierung der Verweilzeitverteilung in den Trennbehältern der Neutralisation und Wäsche kann durch entsprechende Einbauten in den Behältern erfolgen.

In **Schritt d)** schließt sich eine Wäsche der organischen Phase mit Waschflüssigkeit, bevorzugt Wasser, an. Das dabei erhaltene Gemisch aus organischen und wässrigen Bestandteilen wird in **Schritt e.1)** in einem Trennbehälter in eine organische Phase enthaltend Di- und Polyamine der Diphenylmethanreihe (**2a**) und eine wässrige Phase aufgetrennt, um Restgehalte an Salz zu entfernen (bevorzugt wie in DE-A-2549890 auf Seite 3 beschrieben). Die Phasentrennung in Schritt e.1) führt dazu, dass der Anteil wässriger Bestandteile in der organischen Phase (**2a**) weniger als 10 Massen-%, bezogen auf die Gesamtmasse von (**2a**), beträgt. Die Gesamtmasse von (**2a**) bezieht sich dabei auf die Summe aller Bestandteile der in Schritt e.1) von der wässrigen Phase abgetrennten organischen Phase, d. h., organische Bestandteile (die den Hauptanteil von (2a) ausmachen), dispergierte wässrige Bestandteile und ggf. mitgerissene Mulmschicht.

In **Schritt e.2**) werden die wässrigen Anteile der in Schritt e.1) erhaltenen organischen Phase (**2a**) weitgehend bis vollständig abgetrennt. Ein zu hoher Wasseranteil in der organischen Phase, der sich durch eine Trübung ("Resttrübe") dieser Phase bemerkbar macht (Dispersion von wässrigen Anteilen in der organischen Phase), und/oder eine mitgeschleppte Mulmschicht, können sich wie oben beschrieben nachteilig auf die weiteren Verfahrensschritte auswirken. Eine Verbesserung der MDA-Produktqualität kann demnach erzielt werden, wenn die wässrigen Bestandteile aus der organischen Produktphase abgetrennt werden können. Dies durch eine zweite einfache Phasentrennung ohne weitere Maßnahmen zu erreichen, ist nicht realistisch. Im stationären Erdschwerefeld könnte dies nur bedingt durch eine technisch nicht sinnvolle Trennzeit ermöglicht werden. Dies würde entsprechend große technische Apparate erfordern. Als Nachteile sind u. a. zu nennen: ein hohes Flüssigkeitsaufkommen, steigende Investitionskosten für Apparate, erforderlicher Aufstellungsplatz, Probleme mit der Gebäudestatik, etc.

Die Abtrennung der wässrigen Bestandteile aus der organischen Phase (**2a**) erfolgt erfindungsgemäß durch Einsatz einer Koaleszenz-Hilfe. Erfindungsgemäß wird als Koaleszenz-Hilfe ein Filterbett aus Koaleszenz-Faser-Material verwendet. Die Wahl des Faser-Materials ist unter anderem abhängig von:
- Benetzungseigenschaften der dispersen Phase (Tropfen) auf dem Fasermaterial,
- die Grenzflächenspannung des Stoffsystems,
- der Viskosität beider Phasen des Stoffsystems.

Die feindispersen wässrigen Tröpfchen müssen die Oberfläche des Fasermaterials benetzten können.

Beim Durchtritt der Flüssig-flüssig-Dispersion (wässrige Bestandteile dispergiert in der organischen, MDA enthaltenden Phase (**2a**)) durch das Fasermaterial vermögen die feindispers vorliegenden wässrigen Tröpfchen die Faseroberfläche zu benetzen. Die wässrigen Tröpfchen sammeln sich auf den Fasern an (Tropfen-Faser-Koaleszenz), nach weiterer Faserbelegung verringern sich die Abstände zwischen den angelagerten kleinen Tröpfchen, und schließlich vereinigen sich die Tröpfchen zu größeren Tropfen (Tropfen-Tropfen-Koaleszenz). Bei Überschreiten eines charakteristischen Grenztropfendurchmessers (abhängig vom Stoffsystem, Viskosität, Strömungsbedingungen) werden die nun vergrößerten Tropfen durch die Strömungskräfte im Faserbett abgelöst und treten als deutlich vergrößerte Tropfen im Vergleich zum Eintrittstropfen aus dem Fasermaterial heraus. Aufgrund der verbesserten Sedimentationseigenschaften können diese wässrigen Tropfen in der sich anschließenden Phasentrennung im Erdschwerefeld abgeschieden werden, was zu einer Minimierung der Resttrübe in der MDA-Produktphase und zu einer Verbesserung der Produktqualität führt.

Der Erfolg bei der Trennaufgabe hängt davon ab, dass eine Bildung von Gasblasen vermieden wird, was eine Prozessführung bei Temperaturen unter dem Siedepunkt des dispersen Systems und der einzelnen resultierenden Phasen erfordert und die Verwendung von Inertgasen ausschließt. Daher wird die Trennaufgabe für das erfindungsgemäße System (Schritt (e.2 (i)) im Temperaturbereich von 50 °C bis 120 °C, bevorzugt von 70 °C bis 115 °C und besonders bevorzugt von 75 °C bis 110 °C durchgeführt. Für die Trennaufgabe wird der Druck im Trennsystem so gewählt, dass ein Sieden des dispersen Systems unterbleibt. Der einzustellende Mindestdruck hängt vom Temperaturniveau und der Zusammensetzung des dispersen Systems ab und kann durch einfache Versuche ermittelt werden. Bevorzugt wird die Trennaufgabe bei Atmosphärendruck bis zu einem erhöhten Druck von 10 bar absolut, bevorzugt bis zu 5 bar absolut, besonders bevorzugt bis zu 2 bar absolut, durchgeführt.

Der Faserdurchmesser des Koaleszenzmaterials beträgt bevorzugt von 1,0 µm bis 150 µm, besonders bevorzugt von 1,0 µm bis 100 µm, ganz besonders bevorzugt von 2,0 µm bis 30 µm, wobei bei einem Faserdurchmesser von 2,0 µm bis 30 µm eine nominelle Porengröße von 5 µm bis 40 µm vorliegt, d. h., die Fasern mit einem Durchmesser von 2,0 µm bis 30 µm sind so hergestellt, dass eine Porengröße von 5 µm bis 40 µm entsteht.

Für die Abtrennung der dispers vorliegenden wässrigen Tröpfchen werden Fasern aus wasserbenetzenden Materialien (z. B. Glas oder Metall), bevorzugt Fasern aus einem metallischem Material, besonders bevorzugt Fasern aus einem metallischem Material, das im alkalischen Medium beständig ist, eingesetzt. Ganz besonders bevorzugt wird ein Material aus Edelstahl eingesetzt.

Die spezifische hydraulische Belastung beim Durchführen der organischen Phase (**2a**) durch das Koaleszenz-Fasermaterial liegt bevorzugt im Bereich 1,0 m³/(m²h) bis 10 m³/(m²h), besonders bevorzugt 1,0 m³/(m²h) bis 8,0 m³/(m²h) und ganz besonders bevorzugt 2,0 m³/(m²h) bis 6,0 m³/(m²h).

Die Stärke des erfindungsgemäßen Filterbettes aus Koaleszenz-Fasermaterial beträgt bevorzugt 1,0 mm bis 100 mm, besonders bevorzugt 1,0 mm bis 50 mm und ganz besonders bevorzugt 1,0 mm bis 30 mm.

In **Schritt f)** wird aus der in Schritt e.2 (ii)) erhaltenen organischen Phase enthaltend Di- und Polyamine der Diphenylmethanreihe destillativ Wasser und Anilin abgetrennt. Dies geschieht bevorzugt wie in EP 1 813 597 B1, insbesondere in den Absätzen [0014] bis [0043], beschrieben. Die in Schritt e.2 (ii)) erhaltene organische Phase hat bevorzugt eine Zusammensetzung, bezogen auf das die Gesamtmasse des Gemisches, von 5 bis 10 Massen-% Wasser, und, je nach Einsatzverhältnissen von Anilin und Formaldehyd, 5 bis 90 Massen-%, bevorzugt 5 bis 45 Massen-% Anilin und 5 bis 90 Massen-%, bevorzugt 50 bis 90 Massen-% Di- und Polyamine der Diphenylmethanreihe. Nach Austritt aus der Phasentrennung in Schritt e.2) hat die organische Phase enthaltend Di- und Polyamine üblicherweise eine Temperatur von 50 °C bis 150 °C, bevorzugt von 50 °C bis 120 °C, besonders bevorzugt von 70 °C bis 115 °C und ganz besonders bevorzugt von 75 °C bis 110 °C.

Die so erhaltenen Di- und Polyamine der Diphenylmethanreihe können nach den bekannten Methoden mit Phosgen zu den entsprechenden Di- und Polyisocyanaten der Diphenylmethanreihe umgesetzt werden. Dabei kann die Phosgenierung nach einem der aus dem Stand der Technik bekannten Verfahren durchgeführt werden (z. B. DE-A-844 896 oder DE-A-198 17 691).

Wenn nach dem Trennbehälter der Basenwäsche (Schritt e.1) via Koaleszenz-Trennhilfe der wässrige Anteil in der Roh-MDA enthaltenden organischen Phase minimiert wird, (Schritt e.2) ergeben sich folgende Vorteile:
i) Die Produktqualität wird verbessert, weil mehr Wasser aus der organischen Phase abgetrennt wird und somit weniger Salze in der Roh-MDA enthaltenden organischen Phase verbleiben.
ii) Energiekosten werden eingespart, weil weniger Dampf für die Destillation von Roh-MDA nötig ist, weil durch die verbesserte Phasentrennung weniger Wasser in der Roh-MDA enthaltenden, organischen Phase verbleibt.
iii) Instandhaltungskosten werden eingespart, da das durch Salze verursachte Fouling in nachgelagerten Apparaten (z. B. Verdampfer, Kolonnen) deutlich reduziert wird.

### Beispiele:

### Beispiel 1 (erfindungsgemäß)

Roh-MDA wurde hergestellt (**Schritt a)** des erfindungsgemäßen Verfahrens), indem zunächst Anilin und 30 %iger Formaldehyd (molares Verhältnis 2,1 : 1) bei ca. 95 °C zum Aminal umgesetzt wurden. Nach der Phasentrennung zur Entfernung der wässrigen Phase wurde die organische Phase mit 31 %iger wässriger Salzsäure versetzt (Protonierungsgrad 10 %, d. h., pro mol Aminogruppen wurden 0,1 mol HCl zugegeben) und bei 50 °C bis 150 °C in einer Reaktorkaskade umgesetzt. Nach vollständiger Reaktion wurde das erhaltene Reaktionsgemisch mit 32 %iger Natronlauge im molaren Verhältnis von 1,1 : 1 Natronlauge zu HCl versetzt und in einem Neutralisationsrührbehälter umgesetzt (**Schritt b)** des erfindungsgemäßen Verfahrens). Die Temperatur betrug 115 °C. Der Druck betrug 1,4 bar.

Die neutralisierende Basenmischung wurde anschließend in einem Neutralisationsabscheider in eine wässrige, untere Phase, die zu einem Abwassersammelbehälter geführt wurde, und in eine organische Phase getrennt (**Schritt c)** des erfindungsgemäßen Verfahrens). Die wässrige Phase hatte einen pH-Wert von ca. 13, einen NaCl-Gehalt von ca. 17 Massen-% und eine NaOH-Konzentration von ca. 1,5 Massen-%, jeweils bezogen auf die Gesamtmasse der wässrigen Phase. Die organische, obere Phase, wurde zur Wäsche (**Schritt d)** des erfindungsgemäßen Verfahrens) geleitet. In einem gerührten Waschbehälter wurde das alkalische MDA mit Kondensat gewaschen. Nach Abtrennung des Waschwassers in einem Waschwasserabscheider (**Schritt e.1)** des erfindungsgemäßen Verfahrens) wurde das so erhaltene Roh-MDA in **Schritt e.2)** von weiteren wässrigen Bestandteilen befreit und anschließend in einen Sammelbehälter gepumpt. Das in **Schritt e.2 (ii))** abgetrennte Waschwasser, das einen pH-Wert von ca. 11, einen NaCl-Gehalt von ca. 0,2 Massen-% und eine NaOH-Konzentration von ca. 0,8 Massen-%, jeweils bezogen auf die Gesamtmasse des abgetrennten Waschwassers, hatte, wurde in einen Abwassersammelbehälter gepumpt. Als Koaleszenz-Hilfe wurde in **Schritt e.2 (i))** ein Koaleszenz-Filter aus metallischen Edelstahlfasern (Werkstoff 1.4571) eingesetzt. Dessen nominelle Porengröße betrug 15 µm, und der Faserdurchmesser betrug 10 µm. Es wurden 11 übereinanderliegende, durchströmte Faserfliese benutzt. Die Durchflussgeschwindigkeit betrug 4 m³/(m²h), d. h., 4 m³/h Durchsatz, bezogen auf eine Durchströmquerschittsfläche von 1 m². Die so erhaltene organische Phase war klar und hatte einen Wassergehalt von 7,0 Massen-%. Abschließend wird in **Schritt f)** aus der in **Schritt e.2 (ii))** erhaltenen organischen Phase Wasser und Anilin abdestilliert, wobei als Sumpfprodukt MDA erhalten wird.

### Beispiel 2 (Vergleich)

Die Bedingungen waren die gleichen wie in Beispiel 1, mit der Ausnahme, dass in diesem Beispiel der **Schritt e.2**) nicht durchgeführt wurde. Die so erhaltene organische Phase war trübe und hatte einen Wassergehalt von 7,9 Massen-%.

Ein Vergleich von Beispiel 1 mit Beispiel 2 zeigt, dass durch den Einsatz eines Filterbetts aus Koaleszenz-Faser-Material in **Schritt e.2)** der Wassergehalt und somit anteilig die Verunreinigungen von Restsalzen effektiv reduziert wird, erkennbar daran, dass die organische Phase keine Trübung aufweist und der Wassergehalt gemäß Analyse auf den homogen löslichen Anteil reduziert wird.

## Patentansprüche

1. Verfahren zur Herstellung von Di- und Polyaminen der Diphenylmethanreihe, bei dem
a) Anilin und Formaldehyd in Gegenwart eines sauren Katalysators zu einem Reaktionsgemisch enthaltend Di- und Polyamine der Diphenylmethanreihe umgesetzt werden,
b) das Reaktionsgemisch enthaltend Di- und Polyamine der Diphenylmethanreihe neutralisiert wird,
c) das neutralisierte Reaktionsgemisch enthaltend Di- und Polyamine der Diphenylmethanreihe in einem Trennbehälter in eine organische Phase (**1**) enthaltend Di- und Polyamine der Diphenylmethanreihe und eine wässrige Phase aufgetrennt wird,
d) die organische Phase (**1**) enthaltend Di- und Polyamine der Diphenylmethanreihe in einem Waschbehälter mit Waschflüssigkeit weiter gereinigt wird,
e) das in Schritt d) erhaltene Gemisch von wässrigen Bestandteilen befreit wird, wobei eine organische Phase (**2**) enthaltend Di- und Polyamine der Diphenylmethanreihe erhalten wird,
f) die Di- und Polyamine der Diphenylmethanreihe enthaltende organische Phase (**2**) destillativ von Wasser und Anilin befreit wird,
**dadurch gekennzeichnet, dass**
zur Abtrennung der wässrigen Bestandteile in Schritt e) das in Schritt d) erhaltene Gemisch
e.1) in einem Trennbehälter in eine wässrige und eine organische Phase (**2a**) getrennt wird, wobei in der organischen Phase (**2a**) wässrige Bestandteile dispergiert sind, und dann
e.2) die in Schritt e.1) erhaltene organische Phase (**2a**) (i) bei einer Temperatur im Bereich von 50 °C bis 120 °C und bei einem solchen Druck, dass ein Sieden des dispersen Systems unterbleibt, ohne Verwendung von Inertgasen durch ein Filterbett aus Koaleszenz-Faser-Material geführt und (ii) anschließend in eine wässrige Phase und die organische Phase (**2**) getrennt wird.

2. Verfahren nach Anspruch 1, bei dem die Fasern des Koaleszenz-Faser-Materials aus einem wasserbenetzenden Material gefertigt sind.

3. Verfahren nach Anspruch 2, bei dem das wasserbenetzende Material ausgewählt ist aus Glas oder Metall.

4. Verfahren nach Anspruch 3, bei dem das wasserbenetzende Material Edelstahl ist.

5. Verfahren nach einem der Ansprüche 1 bis 4, bei dem die Stärke des Filterbettes aus Koaleszenz-Fasermaterial 1,0 mm bis 100 mm beträgt.

6. Verfahren zur Herstellung von Di- und Polyisocyanaten der Diphenylmethanreihe umfassend die Herstellung von Di- und Polyaminen der Diphenylmethanreihe gemäß einem Verfahren nach einem der Ansprüche 1 bis 5 und Phosgenierung der so hergestellten Di- und Polyamine der Diphenylmethanreihe.

## Claims

1. Process for the preparation of di- and polyamines of the diphenylmethane series, in which
a) aniline and formaldehyde are reacted in the presence of an acidic catalyst to give a reaction mixture comprising di- and polyamines of the diphenylmethane series,
b) the reaction mixture comprising di- and polyamines of the diphenylmethane series is neutralized,
c) the neutralized reaction mixture comprising di- and polyamines of the diphenylmethane series is separated in a separation container into an organic phase (**1**) comprising di- and polyamines of the diphenylmethane series and an aqueous phase,
d) the organic phase (**1**) comprising di- and polyamines of the diphenylmethane series is further purified in a washing container with washing liquid,
e) the mixture obtained in step d) is freed from aqueous constituents, giving an organic phase (**2**) comprising di- and polyamines of the diphenylmethane series,
f) the organic phase (**2**) comprising di- and polyamines of the diphenylmethane series is freed distillatively from water and aniline,
**characterized in that**
for the separation off of the aqueous constituents in step e), the mixture obtained in step d)
e.1) is separated in a separating container into an aqueous and an organic phase (**2a**)**,** wherein aqueous constituents are dispersed in the organic phase (**2a**), and then
e.2) the organic phase (**2a**) obtained in step e.1) (i) is passed at a temperature in the range from 50°C to 120°C and at a pressure such that boiling of the disperse system does not occur, without use of inert gases, through a filter bed made of coalescence fiber material and (ii) is then separated into an aqueous phase and the organic phase (**2**).

2. Process according to Claim 1, in which the fibers of the coalescence fiber material are prepared from a water-wetting material.

3. Process according to Claim 2, in which the water-wetting material is selected from glass or metal.

4. Process according to Claim 3, in which the water-wetting material is stainless steel.

5. Process according to any one of Claims 1 to 4, in which the thickness of the filter bed of coalescence fiber material is 1.0 mm to 100 mm.

6. Process for the preparation of di- and polyisocyanates of the diphenylmethane series comprising the preparation of di- and polyamines of the diphenylmethane series according to a process according to any one of Claims 1 to 5 and phosgenation of the di-and polyamines of the diphenylmethane series thus prepared.

## Revendications

1. Procédé de fabrication de di- et polyamines de la série du diphénylméthane, selon lequel
a) de l'aniline et du formaldéhyde sont mis en réaction en présence d'un catalyseur acide pour former un mélange réactionnel contenant des di- et polyamines de la série du diphénylméthane,
b) le mélange réactionnel contenant les di- et polyamines de la série du diphénylméthane est neutralisé,
c) le mélange réactionnel neutralisé contenant les di- et polyamines de la série du diphénylméthane est séparé dans un contenant de séparation en une phase organique (1) contenant les di- et polyamines de la série du diphénylméthane et une phase aqueuse,
d) la phase organique (1) contenant les di- et polyamines de la série du diphénylméthane est davantage purifiée dans un contenant de lavage avec un liquide de lavage,
e) le mélange obtenu à l'étape d) est débarrassé des constituants aqueux, une phase organique (2) contenant les di- et polyamines de la série du diphénylméthane étant obtenue,
f) la phase organique (2) contenant les di- et polyamines de la série du diphénylméthane est débarrassée par distillation de l'eau et de l'aniline,
**caractérisé en ce que**
pour la séparation des constituants aqueux à l'étape e), le mélange obtenu à l'étape d)
e.1) est séparé dans un contenant de séparation en une phase aqueuse et une phase organique (2a), des constituants aqueux étant dispersés dans la phase organique (2a), puis
e.2) la phase organique (2a) obtenue à l'étape e.1) (i) est acheminée à une température dans la plage allant de 50 °C à 120 °C et à une pression telle qu'une ébullition du système dispersé n'ait pas lieu, sans utiliser de gaz inertes, au travers d'un lit de filtration en un matériau fibreux de coalescence, puis (ii) est séparée en une phase aqueuse et la phase organique (2).

2. Procédé selon la revendication 1, selon lequel les fibres du matériau fibreux de coalescence sont fabriquées en un matériau mouillé par l'eau.

3. Procédé selon la revendication 2, selon lequel le matériau mouillé par l'eau est choisi parmi le verre ou le métal.

4. Procédé selon la revendication 3, selon lequel le matériau mouillé par l'eau est l'acier inoxydable.

5. Procédé selon l'une quelconque des revendications 1 à 4, selon lequel l'épaisseur du lit de filtration en le matériau fibreux de coalescence est de 1,0 mm à 100 mm.

6. Procédé de fabrication de di- et polyisocyanates de la série du diphénylméthane, comprenant la fabrication de di- et polyamines de la série du diphénylméthane par un procédé selon l'une quelconque des revendications 1 à 5 et la phosgénation des di- et polyamines de la série du diphénylméthane ainsi fabriquées.
